# EUROPEAN PATENT SPECIFICATION

(11) **EP 0 495 418 B1**
(45) Date of publication and mention of the grant of the patent: **15.10.1997**
(21) Application number: 92100398.4
(22) Date of filing: 12.01.1992
(51) Int. Cl.: C07C 273/04, B01J 10/00, B01J 19/24

(54) **Process and device for increasing the yield and the production potential of urea reactors**
Verfahren und Vorrichtung zur Verbesserung der Ausbeute und Herstellungspotential von Harnstoffreaktoren
Procédé et dispositif pour augmenter le rendement et le potentiel de production des réacteurs d'urée

(30) Priority: 15.01.1991 CH 103/91
(43) Date of publication of application: 22.07.1992
(73) Proprietor: UREA CASALE S.A., CH-6900 Lugano-Besso (CH)
(72) Inventor: Dente, Mario, I-20154 Milan (IT); Bozzano, Sergio, I-20057 Milan (IT)
(74) Representative: Bottero, Claudio

(56) References cited:
- EP-A- 0 011 976
- CH-A- 344 716
- US-A- 3 049 563
- US-A- 3 222 040

## Description

The present invention relates to a method of producing urea comprising the steps of:
- reacting ammonia and carbon dioxide at high pressure and temperature within a synthesis reactor by cocurrently upwardly flowing a liquid phase including ammonia and a gas phase including carbon dioxide,
- separating the liquid phase from the gas phase by means of a plurality of baffles creating a plurality of compartments within the reactor for avoiding excessive mixing of the liquid phase and to redistribute the gas phase in bubbles of a size suitable for increasing heat and mass transfer between the phases.

The invention also relates to a device for carrying out said method, as well as to a reactor for producing urea from ammonia and carbon dioxide at high pressure and temperature.

### Description of the Known Art.

In modern reactors for the synthesis of urea, the cylindrical shell of the reactor under pressure, inside which two phases, a gas one and a liquid one, flow cocurrently, is divided into compartments by perforated plates. The purpose of this configuration is to avoid the excessive mixing of the entire liquid phase contained in the reactor, which would tend to turn it into a complete mixing reactor, thus reducing the urea yield: by dividing the reactor into several stages by means of plates the amount of mixing is reduced, and the behaviour of the liquid phase is brought closer to the behaviour of a piston flow reactor, which is notoriously the most favourable for keeping the urea yield relatively high.

By dividing the reactor into stages by means of perforated plates, it is also possible to redistribute the gas, flowing upwards along the column, intermittently in smaller bubbles more suitable for increasing heat and matter exchange between the two phases. In effect, the rising showers of bubbles are subjected to coalescence phenomena which progressively increase the size of the bubbles, thus reducing the exchange surface between phases: this negative phenomenon is partly compensated by the redistribution brought about by the perforated flat plates.

However, the cocurrent flow of gas and liquid over each perforated plate produces some adverse effects on both the heat and matter exchange and the urea yield (the latter owing to a diminution of the flow of reagents into the liquid phase, as well as to the reduction in temperature because of the smaller esothermic reaction and also to the reduction in the liquid hold-up in the reactor). In effect, the vapours and liquid cannot go through the perforations in the flat baffles simultaneously, but are forced to do so alternately by means of forming showers of steam bubbles, separated by liquid pistons in continuous phase. Such an arrangement, as compared with a uniform distribution of the bubbles, with the same amount of vapours brings about a higher concentration of bubbles inside the showers alternating with the liquid pistons. The result is a significant increase in coalescence of the bubbles between one plate and the next, increasing their average size, a reduction of the vapour/liquid surface, and a worsening of the gas phase/liquid phase exchange; since less vapour is exchanged with the liquid phase, the volume available for this phase is also reduced (and the temperature it has reached is also lowered). Moreover, between the plates and the cylinder there is in general a circular slit through which part of the vapours may go with less exchange efficiency. Altogether, these causes reduce the urea yield, compared with the yield obtainable with a uniform distribution, instead of the alternating one, of the gas bubbles after each perforated plate.

Another limitative aspect of the State of the Art concerns the possibility of increasing the production potential in reactors in existing plants. Generally the reactor, owing to its potential liquid phase capacity, is very large indeed compared to the nominal urea production required of it, and this fact would lend itself, in principle, to possible increases in production with an almost constant urea yield. However, the bad distribution of gas, the size of the bubbles owing to parasitical coalescence, the mass and heat exchange between phases, the effective volume left to the liquid phase (in which the reaction forming the urea takes place), urea yield, drastically worsen as the gas and liquid capacity increases, so that urea production does not increase proportionately to the increase in total liquid and gas capacity.

US 3,222,040 concerns a sectional support plate for a packed tower, wherein countercurrently flowing gas and liquid phases are contacted, having such structural features whereby the weight of the packing elements which the section can carry is materially increased.

According to this reference, this sectional support plate comprises an upper portion, which is generally arched in cross-section, having walls perforated at least in the upper part thereof, as well as a perforated base portion provided with down-turned outer edges.

EP 0 011 976 discloses, on the other hand, a baffle system for use on a plate for mass transfer operations in fractionating columns, wherein countercurrently flowing gas and liquid phases are contacted.

According to this reference, such a baffle system comprises a channel through which a two-phase mixture of liquid and vapour flows, the surface of the channel having a plurality of holes for allowing escape of the vapour, each hole being provided with a deflector for deflecting the two-phase mixture towards one end of the channel.

Finally, US 3,049,563 discloses a process and a reactor for the synthesis of urea capable of achieving high urea yields without resorting to excessive pressures.

According to this reference, ammonia and carbon dioxide are reacted within a reaction zone defined by a vertically extended column which may contain bubble trays or perforated plates or other suitable means, such as various known forms of column packing, all of which serve to effect division and intimate contact of the fluids passing through the column.

### SUMMARY OF THE INVENTION

The main purpose of this invention is to provide a method of producing urea which allows to eliminate the above-mentioned drawbacks and to increase the synthesis reaction yield and the potential of urea reactors.

This purpose is achieved, according to the invention, by a method as defined in appended claim 1.

Another purpose of the invention is to provide particularly simple and efficient devices to carry out the method in question. The latter is now characterized by the fact that in every transfer from one compartment into the other the gas and liquid phases are made to flow in mutually separate and distributed ways, each with a continuous, permanent and even flow.

In a preferred embodiment, the working device is characterized by the fact that the perforations in the plates have dimensional and/or shape gradients, creating area fractions which permit the flow of liquid in the substantial absence of gas bubbles, respectively the sliding and evenly distributed concentration of gas bubbles in the area which would be hard to reach by the liquid.

Therefore, according to an aspect of this invention, the reactor's perforated plates are made in such a way as to allow the more even distribution, with a permanent flow, of the gas bubbles avoiding their coalescing between a plate and the next and the adverse effects of the two-phase movement with showers of bubbles alternating with a continuous liquid flow. The result is an increase in urea yield and of the reactor's production potential.

In a particularly simple and efficient and therefore preferred embodiment, the plates are differently perforated and shaped in such a way as to allow the continuous and permanent flow of both the gas and the liquid, both flowing along routes which are mutually separate and distributed through each plate. The size of the perforations is different for the fractions of area of the plate intended respectively for the flow of liquid and the flow of gas; the size' of the perforations in the zones intended for the flow of liquid being such as to prevent the passage through them of gas bubbles together with the liquid, but rather to favour their sliding towards zones intended for the gas flow. The fractions of area of the shaped zones intended for the flow of the two phases are distributed in such a way as to ensure the even distribution of the gas bubbles formed through said shaped perforated plates.

The various aspects and advantages of the invention will be made more clear by the description of the embodiment represented in the drawings in which:
- Fig. 1 shows the schematic and partial longitudinal section of a multi-compartment reactor;
- Fig. 2 is an enlarged scale cross-sectional view of the elements forming the plates, comprised in circle A in fig. 1;
- Fig. 3 is an overhand view of the upper part 2 of an element ELi;
- Fig. 3A shows a planar development of the section taken along the line A-A of Fig. 4 of a rectangular ELi element;
- Fig. 4 is the front view of a plate SPi formed by rectangular elements EL'i-EL'n;
- Fig. 4A (analogous to fig. 2) is an enlarged view of elements EL'm-1, EL'm, EL'm+1 in fig. 4;
- Figs. 5 and 5A are enlarged views of perforated portions on wall 2 (at right angles to the reactor's axis) respectively on walls 4, 4' parallel to the axis of the reactor.

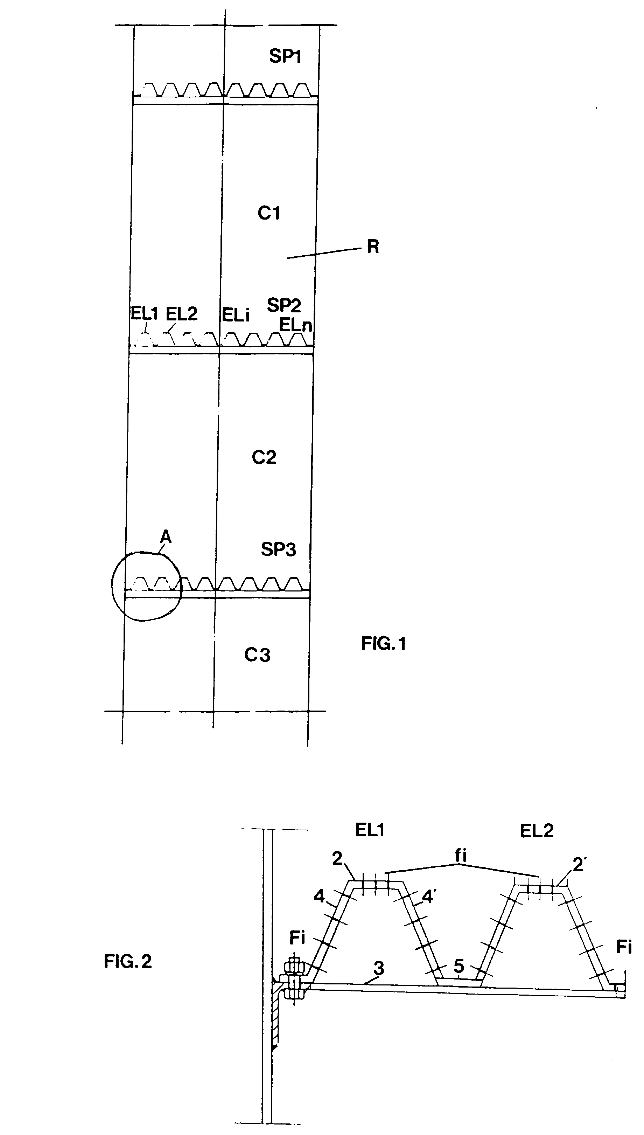

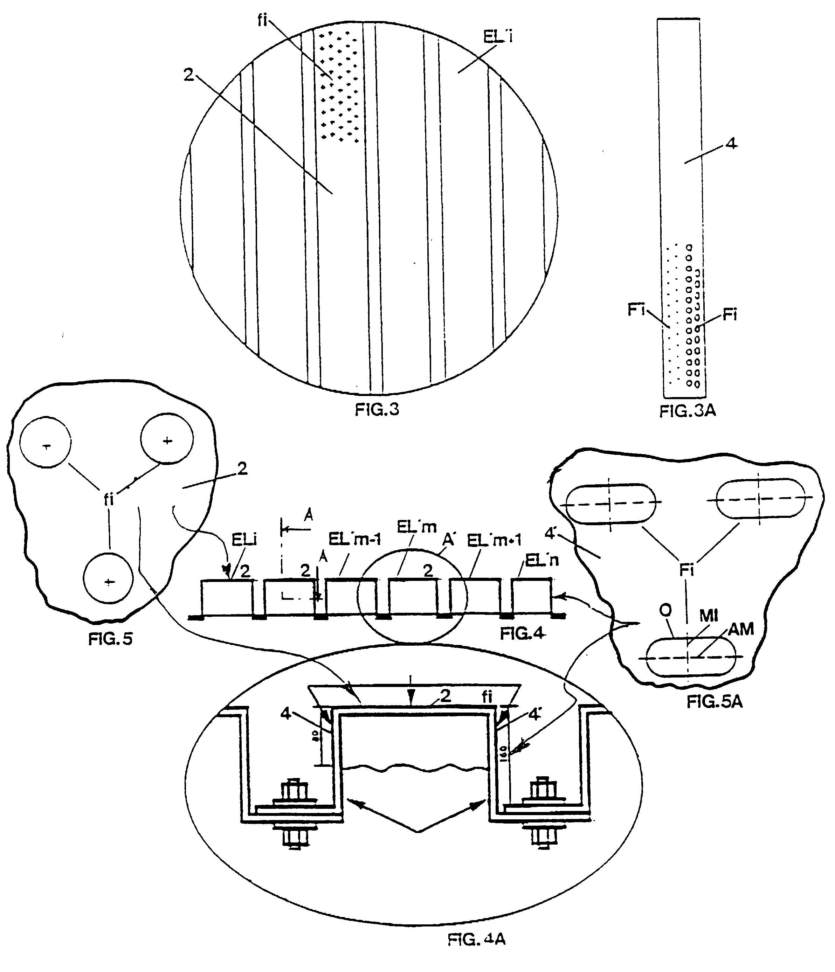

In fig. 1, R shows the central cylindrical shell of the urea reactor and C1, C2, C3 are the three transversal compartments created by the three plates SP1, SP2 and SP3. These are formed by the lozenge shaped elements EL1 .... ELn which in fig. 2 are trapeze-shaped and which in fig. 4 are rectangular by preference.

Fig. 2 shows that every ELi element is shaped like a Greek key, with a wall shaped like an upside down trapeze formed by the side or lesser base 2 at the top, by the greater base at the bottom 3, by the two slanting sides 4 - 4', and by airspace 5. According to the main aspect of the invention, in the embodiment shown in fig. 2, on the two slanting sides 4 and 4' there are perforations Fi larger than perforations fi on wall 2. By preference perforations fi have a diameter of between 1 and 3.5 mm, better still of about 2 to 3 mm, while the large perforations Fi have a diameter which is almost twice that of fi, 2 to 7, Fi perforations being by preference 3 to 6.

Fig. 3 shows an overhand view of a wall 2 of a rectangular or trapeze-shaped element EL'i.

Fig. 4 shows the front view of a flat baffle, perforated and formed by elements from EL'1 to EL'n which are rectangular, i.e. with walls 4 and 4' parallel with the reactor's axis.

Perforations fi on wall 2 are generally circular as in fig. 5; on the other hand, perforations Fi on walls 4 and 4' may be substantially ellipse-shaped as shown in fig. 5A with O. They are characterized by a greater axis AM and by a smaller axis MI.

### EXAMPLE

Operations have been carried out by simulation on a reactor whose model has supplied, under nominal design conditions, for a production of about 1800 t/d, a yield of 64% on the total flow (liquid plus gas): if the yield were to be (improperly) estimated on the sole liquid phase, it would be about 65.5%. By increasing production up to about 2300 t/d, a decrease in yield was noticed. This confirms, indirectly, a) that the reactor is far too big, b) that an improvement in transport processes inside the reactor would not only increase the yield, compared to nominal conditions, but would also improve said yield which would be maintained throughout notable increases of its potential.

Operations were then continued by simulating with a rigorous mathematical model conditions for a daily production of 2300 t in the unmodified reactor. A yield (simulated) was obtained of 61.3% which, if based (improperly) on the sole liquid phase would yield (fictitiously) 63.5%. The absence of a number of data under these conditions does not allow of immediate confirmation, but it would seem that the effective yield predicted by the model under increased production conditions is reasonably close to reality.

Simulation (through the model) of introducing into the reactor new plates also and above all in the section which has not got any at present (i.e., the 16 metres of the lower T.L.) was then carried out. The model coherent with the invention has shown (for the potential of 2300 t/d already achieved) a yield of 65.8% (+4.5% absolute) in respect of the total flow (equal to, improperly, 66.7% on the liquid phase, but with a drastic reduction in steam).

It has also been confirmed that such yield can be maintained for further increases in potential up to 2700 t/d (if that were made possible by the other equipment in the plant). This is a further advantage of the new solution put forward.

Consequently, in theory, under the conditions taken into consideration, an increase in yield (simulated) of 4.5% absolute could be obtained and further increases in production would be tolerable.

Some elements resulting from experiments are given below.

In the description which follows, linear dimensions for baffles and for the passage of liquids are indicative. If necessary for construction purposes, they can be varied by about 5-10%.

This also concerns the number of perforations per m² both where the liquid phase and the gas phase run through.

On the other hand, the size of the perforations for the gas phase must be considered unchangeable while those for the liquid phase are virtually so.

Finally, it is confirmed that the number of perforations per m² for the gas phase and the liquid phase should be read as referred to the areas for the gas phase and the liquid phase and not to the total area (gas plus liquid).

Numbering of the plates (suggested, or actually existing, for that part which has not been replaced) begins at the lower tangential line (T.L.) of the reactor (not shown on drawings).

In the same way, when the plates are being installed they should be rotated alternately (in respect of the dome directrix) by 60°-90°, in so far as fastening points allow.

It has been found that, on the whole, the following instructions should be followed when making the plates:
- the thickness of the metal should not be more than 3.5-4 mm, to permit the punching of the perforations;
- the plates should be oriented alternately, for example fixing them at right angles or at 60°;
- perforations for the installation should be made on that portion of the sheet metal reserved for the passage of liquid;
- perforations per square metre for the surface intended for the passage of gas, should be inderstood as referring to the entire surface of the sheet metal, even that which after being folded becomes lateral (vertical);
- perforations for the gas should be made in equilateral triangular links with a 24 mm pitch;
- after the plate has been shaped it is essential that the baffles are closed at the ends with vertical walls welded to the terminal sections creating a seal to avoid gas escaping from the sides;
- to make up for the thinness suitable supports or stiffeners can be applied to the sheet metal after perforating and shaping;
- the gas should be introduced into the lower part through a horizontal tube with multiple perforations set at right angles to the baffles of the lowest plate: if this were not possible, it would become necessary to arrange two plates, with baffles at right angles between them, close to one another (300+500 mm) which would obtain the same result (since the lower plate would act as distributor for the upper plate).
   The plates should be at a distance of about 2.4 m.
   Perforations for the gas could, in principle, be varied from one plate to the other, with a perforated area decreasing from bottom towards the top: however, with the plates arranged according to the invention, it is possible to maintain even perforations equal to the maximum perforations required for the lower plate. This simplifies construction and installation procedures. In the upper plates, where the vapours (gas) are gradually decreasing this means adjusting the ever increasing level of the liquids inside the domes and a head of gas getting smaller and smaller (part of the perforations intended for the passage of gas will therefore be used by the liquid). In a preferred embodiment;
- perforations for the gas (in the present case) were 2000 holes/(m2 of relative area) with a diameter φ = 3 mm; arranged as an equilateral triangle (preferred arrangement), this means a distance between perforations for example of 24 mm (and a fraction of perforated area, on the area intended for gas, of 1.4%) (fig. 5);
- perforations for the liquid (in the present case), 600 holes/(m2 of relative area) with diameter φ = 8 mm, arranged as an equilateral triangle with a distance of 43 mm. Where oval perforations are used (fig. 5A), their axes are 4 mm - 6 mm, interspersed in the same way (with a fraction of perforated area of about 3% of the area for the liquid).
   Obviously, the metallic strips bearing the perforations, after being folded and installed should be bolted one to another, leaving an edge for the alternating overlap from one dome to the next.

## Claims

1. A method of producing urea comprising the steps of:
- reacting ammonia and carbon dioxide at high pressure and temperature within a synthesis reactor (R) by cocurrently upwardly flowing a liquid phase including ammonia and a gas phase including carbon dioxide,
- separating the liquid phase from the gas phase by means of a plurality of baffles (SP1 - SP3) creating a plurality of compartments (C1 - C3) within the reactor (R) for avoiding excessive mixing of the liquid phase and to redistribute the gas phase in bubbles of a size suitable for increasing heat and mass transfer between the phases;
said method being characterized in that it further comprises the steps of:
a) flowing predominantly the liquid phase through a plurality of first openings (Fi) formed in the side walls (4, 4') of a plurality of elements (EL1 - Eln) open at the bottom and upwardly extending from at least one of said baffles (SP1 - SP3), the first openings (Fi) having a first predetermined size, and
b) flowing predominantly the gas phase through a plurality of second openings (fi) formed in the top wall (2) of said elements (EL1 - Eln), the second openings (fi) having a second predetermined size smaller than the size of said first openings (Fi),
whereby each time the liquid phase and the gas phase pass from one compartment (C1 - C3) into the next they are made to flow through mutually separate routes and are distributed in a substantially even, continuous and permanent flow.

2. A method according to claim 1, characterized in that said elements (EL1 - ELn) have a rectangular cross-section.

3. A method according to claim 1, characterized in that said elements (EL1 - ELn) have a trapezoidal cross-section.

4. A method according to claim 1, characterized in that said first openings (Fi) have a diameter of 2 to 8 mm and said second openings (fi) have a diameter of 1 to 3.5 mm.

5. A method according to claim 4, characterized in that said first openings (Fi) have a diameter of from 3 to 6 mm and said second openings (fi) have a diameter of from 2 to 3 mm.

6. A method according to claim 1, characterized in that said first openings (Fi) are oval-shaped and said second openings (fi) are round.

7. A reactor (R) for producing urea from ammonia and carbon dioxide at high pressure and temperature, said reactor (R) comprising an internal space for containing cocurrent upward flows of a continuous liquid phase including ammonia and a dispersed gas phase including carbon dioxide, said internal space being divided into a plurality of compartments (C1 - C3) by means of a plurality of baffles (SP1 - SP3) to avoid excessive mixing of the liquid phase and to redistribute the gas phase in bubbles of a size suitable for increasing heat and mass transfer between the liquid phase and the gas phase;
characterized in that at least one of said baffles (SP1 - SP3) comprises a plurality of upwardly extending elements (EL1 - Eln), said elements (EL1 - Eln) being open at the bottom and comprising:
i) two perforated side walls (4, 4') comprising a plurality of first openings (Fi) predominantly for flow of the liquid phase, and
ii) a perforated top wall (2) comprising a plurality of second openings (fi) predominantly for flow of the gas phase,
said second openings (fi) in the top wall (2) being smaller in size than the first openings (Fi) in the side walls (4, 4').

8. A reactor (R) according to claim 7, characterized in that said elements (EL1 - ELn) have a rectangular cross-section.

9. A reactor (R) according to claim 7, characterized in that said elements (EL1 - ELn) have a trapezoidal cross-section.

10. A reactor (R) according to claim 7, characterized in that said first openings (Fi) have a diameter of 2 to 8 mm and said second openings (fi) have a diameter of 1 to 3.5 mm.

11. A reactor (R) according to claim 10, characterized in that said first openings (Fi) have a diameter of from 3 to 6 mm and said second openings (fi) have a diameter of from 2 to 3 mm.

12. A reactor (R) according to claim 7, characterized in that said first openings (Fi) are oval-shaped and said second openings (fi) are round.

13. A device for carrying out a method according to anyone of claims 1-6, including a plurality of baffles (SP1 - SP3) insertable within the internal space of a reactor (R) for producing urea, characterized in that at least one of said baffles (SP1 - SP3) comprises a plurality of upwardly extending elements (EL1 - ELn), said elements (EL1 - Eln) being open at the bottom and comprising:
i) two perforated side walls (4, 4') comprising a plurality of first openings (Fi) predominantly for flow of the liquid phase, and
ii) a perforated top wall (2) comprising a plurality of second openings (fi) predominantly for flow of the gas phase,
said second openings (fi) in the top wall (2) being smaller in size than the first openings (Fi) in the side walls (4, 4').

14. A device according to claim 13, characterized in that said elements (EL1 - ELn) have a rectangular cross-section.

15. A device according to claim 13, characterized in that said elements (EL1 - ELn) have a trapezoidal cross-section.

16. A device according to claim 13, characterized in that said first openings (Fi) have a diameter of 2 to 8 mm and said second openings (fi) have a diameter of 1 to 3.5 mm.

17. A device according to claim 16, characterized in that said first openings (Fi) have a diameter of from 3 to 6 mm and said second openings (fi) have a diameter of from 2 to 3 mm.

18. A device according to claim 13, characterized in that said first openings (Fi) are oval-shaped and said second openings (fi) are round.

## Patentansprüche

1. Verfahren zum Herstellen von Harnstoff, welches die folgenden Schritte umfaßt:
- zur Reaktion Bringen von Ammoniak und Kohlendioxid bei hohem Druck und hoher Temperatur in einem Synthesereaktor (R) durch einen nach oben gerichteten Gleichstrom einer flüssigen, Ammoniak enthaltenden Phase und einer Kohlendioxid enthaltenden Gasphase,
- Trennen der flüssigen Phase von der Gasphase mit Hilfe von mehreren Umlenkelementen (SP1-SP3), welche mehrere Abteilungen (C1-C3) in dem Reaktor (R) erzeugen, zum Vermeiden einer übermäßigen Vermischung der flüssigen Phase und zum Umverteilen der Gasphase zu Blasen mit einer Größe, die zur Vergrößerung des Wärme- und Stoffübergangs zwischen den Phasen geeignet ist,
wobei das Verfahren dadurch gekennzeichnet ist, daß es weiterhin die folgenden Schritte umfaßt:
a) Strömenlassen hauptsächlich der flüssigen Phase durch mehrere erste Öffnungen (Fi), welche in den Seitenwänden (4,4') von mehreren Elementen (EL1-ELn) ausgebildet sind, die auf der Unterseite offen sind und sich nach oben von mindestens einem der Umlenkelemente (SP1-SP3) erstrecken, wobei die ersten Öffnungen (Fi) eine erste vorbestimmte Größe aufweisen, und
b) Strömenlassen hauptsächlich der Gasphase durch mehrere zweite Öffnungen (fi), welche in der oberen Wand (2) der Elemente (EL1-ELn) ausgebildet sind, wobei die zweiten Öffnungen (fi) eine zweite vorbestimmte Größe aufweisen, die geringer als die Größe der ersten Öffnungen (Fi) ist,
wodurch jedes Mal, wenn die flüssige Phase und die Gasphase von einer Abteilung (C1-C3) in die nächste übertreten, sie dazu gebracht werden, daß sie auf jeweils verschiedenen Wegen strömen, und in einer im wesentlichen gleichmäßigen, kontinuierlichen und permanenten Strömung verteilt sind.

2. Verfahren nach Anspruch 1, dadurch gekennzeichnet, daß die Elemente (EL1-ELn) einen rechtwinkligen Querschnitt aufweisen.

3. Verfahren nach Anspruch 1, dadurch gekennzeichnet, daß die Elemente (EL1-ELn) einen trapezförmigen Querschnitt aufweisen.

4. Verfahren nach Anspruch 1, dadurch gekennzeichnet, daß die ersten Öffnungen (Fi) einen Durchmesser von 2 bis 8 mm und die zweiten Öffnungen (fi) einen Durchmesser von 1 bis 3,5 mm aufweisen.

5. Verfahren nach Anspruch 4, dadurch gekennzeichnet, daß die ersten Öffnungen (Fi) einen Durchmesser von 3 bis 6 mm und die zweiten Öffnungen (fi) einen Durchmesser von 2 bis 3 mm aufweisen.

6. Verfahren nach Anspruch 1, dadurch gekennzeichnet, daß die ersten Öffnungen (Fi) ovalförmig sind und die zweiten Öffnungen (fi) rund sind.

7. Reaktor (R) zum Erzeugen von Harnstoff aus Ammoniak und Kohlendioxid bei hohem Druck und hoher Temperatur, wobei der Reaktor (R) einen Innenraum zum Aufnehmen eines nach oben gerichteten Gleichstroms einer kontinuierlichen flüssigen Phase, welche Ammoniak enthält, und einer verteilten Gasphase, welche Kohlendioxid enthält, aufweist, wobei der Innenraum in mehrere Abteilungen (C1-C3) mit Hilfe von mehreren Umlenkelementen (SP1-SP3) unterteilt ist, um eine übermäßige Vermischung der flüssigen Phase zu vermeiden und die Gasphase zu Blasen mit einer Größe umzuverteilen, welche für die Vergrößerung des Wärme- und Stoffübergangs zwischen der flüssigen Phase und der Gasphase geeignet ist,
dadurch gekennzeichnet, daß zumindest eines der Umlenkelemente (SP1-SP3) mehrere sich nach oben erstreckende Elemente (EL1-ELn) aufweist, wobei diese Elemente (EL1-ELn) auf der Unterseite offen sind und umfassen:
i) zwei perforierte Seitenwände (4, 4'), welche mehrere erste Öffnungen (Fi) hauptsächlich für eine Strömung der flüssigen Phase umfassen, und
ii) eine perforierte obere Wand (2), welche mehrere zweite Öffnungen (fi) hauptsächlich für eine Strömung der Gasphase umfassen,
wobei die zweiten Öffnungen (fi) in der oberen Wand (2) eine kleinere Größe als die ersten Öffnungen (Fi) in den Seitenwänden (4, 4') aufweisen.

8. Reaktor (R) nach Anspruch 7, dadurch gekennzeichnet, daß die Elemente (EL1-ELn) einen rechtwinkligen Querschnitt aufweisen.

9. Reaktor (R) nach Anspruch 7, dadurch gekennzeichnet, daß die Elemente (EL1-ELn) einen trapezförmigen Querschnitt aufweisen.

10. Reaktor (R) nach Anspruch 7, dadurch gekennzeichnet, daß die ersten Öffnungen (Fi) einen Durchmesser von 2 bis 8 mm und die zweiten Öffnungen (fi) einen Durchmesser von 1 bis 3,5 mm aufweisen.

11. Reaktor (R) nach Anspruch 10, dadurch gekennzeichnet, daß die ersten Öffnungen (Fi) einen Durchmesser von 3 bis 6 mm aufweisen und die zweiten Öffningen (fi) einen Durchmesser von 2 bis 3 mm aufweisen.

12. Reaktor (R) nach Anspruch 7, dadurch gekennzeichnet, daß die ersten Öffnungen (Fi) ovalförmig sind und die zweiten Öffnungen (fi) rund sind.

13. Vorrichtung zum Ausführen eines Verfahrens nach einem der Ansprüche 1 bis 6, welches mehrere Umlenkelemente (SP1-SP3) aufweist, welche in den Innenraum eines Reaktors (R) zum Erzeugen von Harnstoff einfügbar sind, dadurch gekennzeichnet, daß zumindest eines der Umlenkelemente (SP1-SP3) mehrere sich nach oben erstreckende Elemente (EL1-ELn) aufweist, wobei die Elemente (EL1-ELn) auf der Unterseite offen sind und umfassen:
i) zwei perforierte Seitenwände (4, 4'), welche mehrere erste Öffnungen (Fi) hauptsächlich für eine Strömung der flüssigen Phase aufweisen, und
ii) eine perforierte obere Wand (2), welche mehrere zweite Öffnungen (fi) hauptsächlich für eine Strömung der Gasphase aufweisen,
wobei die zweiten Öffnungen (fi) in der oberen Wand (2) eine kleinere Größe als die ersten Öffnungen (Fi) in den Seitenwänden (4, 4') aufweisen.

14. Vorrichtung nach Anspruch 13, dadurch gekennzeichnet, daß die Elemente (EL1-ELn) einen rechtwinkligen Querschnitt aufweisen.

15. Vorrichtung nach Anspruch 13, dadurch gekennzeichnet, daß die Elemente (EL1-ELn) einen trapezförmigen Querschnitt aufweisen.

16. Vorrichtung nach Anspruch 13, dadurch gekennzeichnet, daß die ersten Öffnungen (Fi) einen Durchmesser von 2 bis 8 mm und die zweiten Öffnungen (fi) einen Durchmesser von 1 bis 3,5 mm aufweisen.

17. Vorrichtung nach Anspruch 16, dadurch gekennzeichnet, daß die ersten Öffnungen (Fi) einen Durchmesser von 3 bis 6 mm und die zweiten Öffnungen (fi) einen Durchmesser von 2 bis 3 mm aufweisen.

18. Vorrichtung nach Anspruch 13, dadurch gekennzeichnet, daß die ersten Öffnungen (Fi) ovalförmig sind und die zweiten Öffnungen (fi) rund sind.

## Revendications

1. Procédé de production d'urée comprenant les étapes consistant à :
- faire réagir de l'ammoniaque et du dioxyde de carbone à pression et température élevée dans un réacteur de synthèse (R) en faisant s'écouler en co-courants ascendants, une phase liquide incluant de l'ammoniaque et une phase gazeuse incluant du dioxyde de carbone,
- séparer la phase liquide de la phase gazeuse au moyen d'une pluralité de déflecteurs (SP1-SP3) formant une pluralité de compartiments (C1-C3) dans le réacteur (R) pour éviter un mélange excessif de la phase liquide et pour redistribuer la phase gazeuse sous forme de bulles d'une taille appropriée pour augmenter le transfert de chaleur et de matière entre les phases ;
ledit procédé étant caractérisé en ce qu'il comprend en outre les étapes consistant à :
- faire s'écouler principalement la phase liquide à travers une pluralité de premières ouvertures (Fi), formées dans les parois latérales (4, 4') d'une pluralité d'éléments (EL1-ELn) ouverts au fond et s'étendant vers le haut depuis au moins l'un desdits déflecteurs (SP1-SP3), les premières ouvertures (Fi) ayant une première taille prédéterminée, et
- faire s'écouler principalement la phase gazeuse à travers une pluralité de secondes ouvertures (fi), formées dans la paroi supérieure (2) desdits éléments (EL1-ELn), les secondes ouvertures (fi) ayant une seconde taille prédéterminée plus petite que la taille desdites premières ouvertures (Fi),
de sorte qu'à chaque fois que la phase liquide et la phase gazeuse passent d'un compartiment (C1-C3) dans le suivant, elles sont forcées de s'écouler à travers des voies mutuellement séparées et sont distribués selon un écoulement essentiellement régulier, continu et permanent.

2. Procédé selon la revendication 1, caractérisé en ce que lesdits éléments (EL1-ELn) ont une section en coupe rectangulaire.

3. Procédé selon la revendication 1, caractérisé en ce que lesdits éléments (EL1-ELn) ont une section en coupe trapézoïdale.

4. Procédé selon la revendication 1, caractérisé en ce que lesdites premières ouvertures (Fi) ont un diamètre de 2 à 8 mm et en ce que lesdites secondes ouvertures (fi) ont un diamètre de 1 à 3,5 mm.

5. Procédé selon la revendication 4, caractérisé en ce que lesdites premières ouvertures (Fi) ont un diamètre de 3 à 6 mm et en ce que lesdites secondes ouvertures (fi) ont un diamètre de 2 à 3 mm.

6. Procédé selon la revendication 1, caractérisé en ce que lesdites premières ouvertures (Fi) sont de forme ovale et lesdites secondes ouvertures (fi) sont circulaires.

7. Réacteur (R) pour produire de l'urée à partir d'ammoniaque et de dioxyde de carbone à pression et température élevées, ledit réacteur (R) comprenant un espace interne pour contenir des écoulements en co-courants ascendants, d'une phase liquide continue incluant de l'ammoniaque et d'une phase gazeuse dispersée incluant du dioxyde de carbone, ledit espace interne étant divisé en une pluralité de compartiments (C1-C3) au moyen d'une pluralité de déflecteurs (SP1-SP3) pour éviter un mélange excessif de la phase liquide et pour redistribuer la phase gazeuse sous forme de bulles d'une taille appropriée pour augmenter le transfert de chaleur et de matière entre la phase liquide et la phase gazeuse ;
caractérisé en ce qu'au moins l'un desdits déflecteurs (SP1-SP3) comprend une pluralité d'éléments s'étendant vers le haut (EL1-ELn) ouverts au fond et comprenant :
i) deux parois latérales perforées (4, 4') comprenant une pluralité de premières ouvertures (Fi) principalement pour l'écoulement de la phase liquide, et
ii) une paroi supérieure perforée (2) comprenant une pluralité de secondes ouvertures (fi) principalement pour l'écoulement de la phase gazeuse,
lesdites secondes ouvertures (fi) dans la paroi supérieure (2) étant d'une taille plus petite que les premières ouvertures (Fi) dans les parois latérales (4,4').

8. Réacteur (R) selon la revendication 7, caractérisé en ce que lesdits éléments (EL1-ELn) ont une section en coupe rectangulaire.

9. Réacteur (R) selon la revendication 7, caractérisé en ce que lesdits éléments (EL1-ELn) ont une section en coupe trapézoïdale.

10. Réacteur (R) selon la revendication 7, caractérisé en ce que lesdites premières ouvertures (Fi) ont un diamètre de 2 à 8 mm et en ce que lesdites secondes ouvertures (fi) ont un diamètre de 1 à 3,5 mm.

11. Réacteur (R) selon la revendication 10, caractérisé en ce que lesdites premières ouvertures (Fi) ont un diamètre de 3 à 6 mm et en ce que lesdites secondes ouvertures (fi) ont un diamètre de 2 à 3 mm.

12. Procédé selon la revendication 7, caractérisé en ce que lesdites premières ouvertures (Fi) sont de forme ovale et lesdites secondes ouvertures (fi) sont circulaires.

13. Dispositif pour effectuer un procédé selon l'une quelconque des revendications 1 à 6, incluant une pluralité de déflecteurs (SP1-SP3) aptes à être insérés dans l'espace interne d'un réacteur (R) pour produire de l'urée, caractérisé en ce que au moins l'un desdits déflecteurs (SP1-SP3) comprend une pluralité d'éléments s'étendant vers le haut (EL1-ELn), lesdits éléments (El1-ELn) étant ouverts au fond et comprenant :
i) deux parois latérales perforées (4, 4') comprenant une pluralité de premières ouvertures (Fi) principalement pour l'écoulement de la phase liquide, et
ii) une paroi supérieure perforée (2) comprenant une pluralité de secondes ouvertures (fi) principalement pour l'écoulement de la phase gazeuse,
lesdites secondes ouvertures (fi) dans la paroi supérieure (2) étant d'une taille plus petite que les premières ouvertures (Fi) dans les parois latérales (4,4').

14. Dispositif selon la revendication 13, caractérisé en ce que lesdits éléments (EL1-ELn) ont une section en coupe rectangulaire.

15. Dispositif selon la revendication 13, caractérisé en ce que lesdits éléments (EL1-ELn) ont une section en coupe trapézoïdale.

16. Dispositif selon la revendication 13, caractérisé en ce que lesdites premières ouvertures (Fi) ont un diamètre de 2 à 8 mm et en ce que lesdites secondes ouvertures (fi) ont un diamètre de 1 à 3,5 mm.

17. Dispositif selon la revendication 16, caractérisé en ce que lesdites premières ouvertures (Fi) ont un diamètre de 3 à 6 mm et en ce que lesdites secondes ouvertures (fi) ont un diamètre de 2 à 3 mm.

18. Procédé selon la revendication 13, caractérisé en ce que lesdites premières ouvertures (Fi) sont de forme ovale et lesdites secondes ouvertures (fi) sont circulaires.
